# FASCICULE DE BREVET EUROPEEN

(11) **EP 1 203 789 B1**
(45) Date de publication et mention de la délivrance du brevet: **15.06.2005**
(21) Numéro de dépôt: 01402714.8
(22) Date de dépôt: 19.10.2001
(51) Int. Cl.: C08L 33/26, C08L 5/00, A61K 7/48

(54) **Composition contenant des silicates mixtes, polysaccharide et polymère réticule et ses utilisations**
Zusammensetzung enthaltend ein gemischtes Silikat, Polysaccharid und vernetztes Polymer und seine Verwendung
Composition comprising a mixed silicate, polysacharide and crosslinked polymer and its utilisation

(30) Priorité: 06.11.2000 FR 0014175
(43) Date de publication de la demande: 08.05.2002
(73) Titulaire: L'OREAL, 75008 Paris (FR)
(72) Inventeur: Chevalier, Véronique, 94440 Villecresnes (FR); Hurel, Valérie, 91190 Gif S/Yvette (FR)
(74) Mandataire: Kromer, Christophe

(56) Documents cités:
- EP-A- 0 205 744
- US-A- 5 112 391

## Description

La présente invention concerne une composition contenant un silicate mixte, un polysaccharide et un polymère poly(acide 2-acrylamido 2-méthylpropane sulfonique) réticulé, et aux utilisations de ladite composition, en particulier dans le domaine cosmétique comme agent tenseur, notamment pour diminuer et/ou à effacer les rides et/ou les ridules de la peau.

Il est connu d'utiliser des argiles et en particulier des silicates mixtes, dans les compositions cosmétiques. Ces silicates sont connus pour leurs propriétés thixotropes et peuvent être utilisés en tant qu'agents épaississants, filmogènes, émulsifiants, adsorbants et/ou absorbants. Ce sont d'excellents gélifiants résistant à des conditions extrêmes de pH et de température, et leurs domaines d'application en dehors du domaine cosmétique sont très vastes (par exemple peintures, agriculture, produits de bâtiment).

Le brevet WO-A-97/31619 décrit des compositions cosmétiques nettoyantes et astringentes renfermant, entre autres, de la laponite qui appartient à la famille des silicates mixtes et plus particulièrement à la classe des silicates de sodium et magnésium. Les laponites sont des silicates colloïdaux stratifiés de synthèse, utilisés pour leurs propriétés d'absorption d'huile. Une telle laponite peut être utilisée en tant qu'agent épaississant et/ou gélifiant dans les compositions cosmétiques comme décrit dans la demande de brevet EP-A-412 449.

En outre, les silicates mixtes tels que la laponite sont décrits comme agent tenseur dans le document EP-A-1 008 340.

Malheureusement, les compositions contenant des silicates mixtes, tout particulièrement lorsqu'elles sont sous forme d'émulsion (composition comprenant au moins une phase aqueuse et au moins une phase huileuse dispersée l'une dans l'autre) et lorsqu'elles contiennent plus de 3 % de silicates mixtes, présentent une stabilité insuffisante, ce qui rend difficile l'incorporation d'une telle quantité de ces silicates dans des compositions cosmétiques, car ces dernières doivent présenter une excellente stabilité pendant toute leur durée de vie commerciale. L'instabilité des émulsions contenant plus de 3 % en poids de silicates mixtes tels que la laponite se produit au bout de quelques jours voire une à deux semaines, et elle se traduit entre autres par un déphasage des phases huileuse et aqueuse, par une évolution de la couleur et/ou de l'odeur, et par un changement de viscosité au cours du temps de stockage.

La demanderesse a découvert de manière surprenante que le fait d'associer aux silicates mixtes, un polysaccharide et un polymère dérivé d'acide 2-acrylamido 2-méthylpropane sulfonique, permettait d'obtenir des compositions, et plus particulièrement des émulsions, présentant une très bonne stabilité dans le temps.

Le document EP-A-1 008 340 décrit l'association de laponite et de gomme de xanthane. Toutefois, au-delà de 3 % de laponite, la composition contenant cette association n'a pas une stabilité suffisante pour constituer un produit commercialement viable.

La présente invention a donc pour objet une composition contenant au moins un silicate mixte, au moins un polysaccharide et au moins un polymère poly(acide 2-acrylamido 2-méthylpropane sulfonique) réticulé et neutralisé à au moins 90 %.

La composition selon l'invention a l'avantage d'être particulièrement stable, même quand la quantité de silicate mixte dépasse 3 % en poids, et même lorsqu'elle se présente sous forme d'émulsion, notamment sous forme d'émulsion huile-dans-eau. Elle reste tout à fait stable à toutes températures après deux mois de stockage ou plus, c'est-à-dire que macroscopiquement, on n'observe ni changement de couleur, d'odeur, ni remontée d'huile (déphasage), ni changement de viscosité, ni variation de pH, et que, microscopiquement, on n'observe aucun changement d'apparence.

Selon un mode préféré de réalisation de l'invention, la composition de l'invention constitue une composition cosmétique et/ou dermatologique et comprend un milieu physiologiquement acceptable. On entend ici par « milieu physiologiquement acceptable » un milieu compatible avec la peau y compris le cuir chevelu, les lèvres et les cheveux.

On entend par silicate mixte dans la présente invention, tous les silicates d'origine naturelle ou synthétique renfermant plusieurs (deux ou plus) types de cations choisis parmi les métaux alcalins (par exemple Na, Li, K) ou alcalino-terreux (par exemple Be, Mg, Ca), les métaux de transition et l'aluminium.

Ces silicates mixtes peuvent être d'origine naturelle ou synthétique, et peuvent être choisis par exemple parmi les montmorillonites, les hectorites, les bentonites, la beidellite, les saponites. Selon un mode préféré de réalisation de l'invention, les silicates mixtes utilisés sont plus particulièrement choisis parmi les hectorites et les bentonites, et encore mieux parmi les laponites.

Pour garantir de bonnes propriétés cosmétiques, ces silicates doivent se présenter de préférence sous une forme finement divisée, et en particulier sous forme de particules ayant une taille moyenne allant de 5 nm à 1 µm (de 5 à 1000 nm), et de préférence de 20 nm à 600 nm. Les particules de silicates mixtes ont généralement la forme de disques ou de feuillets. Aussi, on entend ici par taille moyenne de particules, la taille moyenne en nombre de la plus grande dimension (longueur) de ces disques ou feuillets. Ces particules sous forme de disques ou feuillets ont généralement une épaisseur allant d'environ 0,5 à 5 nm.

On utilise de préférence dans la présente invention des phyllosilicates, à savoir des silicates ayant une structure dans laquelle les tétraèdres SiO4 sont organisés en feuillets entre lesquels se trouvent enfermés les cations métalliques.

Une famille de silicates particulièrement préférée dans les compositions de la présente invention est celle des laponites. Les laponites sont des silicates de magnésium, de sodium et éventuellement de lithium, ayant une structure en couches semblable à celle des montmorillonites. La laponite est la forme synthétique du minéral naturel appelé "hectorite". L'origine synthétique de cette famille de silicates présente un avantage considérable par rapport à la forme naturelle car elle permet une bonne maîtrise de la composition du produit. En outre, les laponites ont l'avantage d'avoir une taille de particules bien inférieure à celles de l'hectorite et de la bentonite naturelles.

Comme laponites, on peut citer notamment les produits vendus par la société Laporte sous le nom Laponite XLS, Laponite XLG, Laponite RD, Laponite RDS (ces produits sont des silicates de sodium et de magnésium et des silicates de sodium, de lithium et de magnésium).

Comme silicates mixtes, on peut citer aussi les bentonites comme le produit vendu sous la dénomination Bentone HC par la société RHEOX ; les silicates de magnésium et d'aluminium notamment hydratés comme le produit vendu par la société Vanderbilt Company sous le nom Veegum ultra, ou encore les silicates de calcium et notamment celui sous forme synthétique vendu par la société Celite sous le nom de Micro-cel C.

La quantité de silicate(s) mixte(s) dans la composition de l'invention peut aller par exemple de 0,01 à 10 % en poids, de préférence de 0,05 à 5 % en poids et mieux de 1 à 5 % en poids par rapport au poids total de la composition.

Comme polysaccharides utilisables dans la composition de l'invention, on peut citer plus particulièrement les dérivés cellulosiques ; les galactomannanes (polysaccharides composés principalement d'unités galactose et mannose) et leurs dérivés, telles que la gomme de guar, la gomme de caroube, et les gommes de guar modifiées notamment par greffage de groupement alkyle ; les extraits d'algues tels que l'agar-agar, les carraghénanes, les alginates ; les exsudats de plantes tels que la gomme arabique, la gomme karaya, la gomme adragante et la gomme de ghatti ; les exsudats de micro-organismes tels que la gomme de xanthane, la gomme de gellane, la gomme de rhamsan ; et leurs mélanges. Selon un mode préféré de réalisation de l'invention, le polysaccharide utilisé est une gomme et en particulier la gomme de xanthane.

La quantité de polysaccharide(s) dans la composition de l'invention va de préférence de 0,01 à 8 % en poids, de préférence de 0,05 à 5 % en poids et mieux de 0,1 à 3 % en poids par rapport au poids total de la composition.

Dans la présente demande, on entend par « polymère poly(acide 2-acrylamido 2-méthylpropane sulfonique) réticulé et neutralisé à au moins 90 % » un homopolymère dérivé de l'acide 2-acrylamido 2-méthylpropane sulfonique, qui est réticulé et qui est pratiquement totalement neutralisé ou totalement neutralisé. Ces polymères sont hydrosolubles ou gonflables dans l'eau.

Les polymères utilisés dans la composition de l'invention sont en général caractérisés par le fait qu'ils comprennent, distribués de façon aléatoire :
a) de 90 à 99,9% en poids de motifs de formule générale (I) suivante : 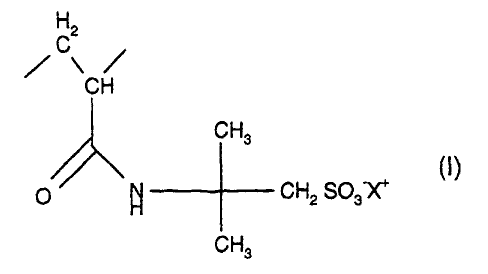 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.

De façon préférentielle, les polymères de l'invention comportent un nombre de motifs de formule (I) dans une quantité suffisamment élevée pour obtenir des particules de polymère dont le volume hydrodynamique en solution d'eau présente un rayon allant de 10 à 500 nm et dont la distribution est homogène et unimodale.

Les polymères utilisés dans la composition selon l'invention plus particulièrement préférés comprennent de 98 à 99,5 % en poids de motifs de formule (I) et de 0,5 à 2 % en poids de motifs réticulants.

X⁺ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations NH₄⁺ et 0 à 10% mole sont des protons (H⁺).

Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylene-bis-acrylamide ou le divinylbenzène.

Les monomères de réticulation ayant au moins deux doubles liaisons oléfiniques sont plus particulièrement choisis parmi ceux répondant à la formule générale (II) suivante : 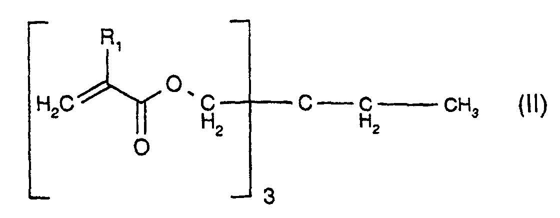 dans laquelle R₁ désigne un atome d'hydrogène ou un alkyle en C₁-C₄ et plus particulièrement méthyle. Le monomère de réticulation est de préférence le triméthylol propane triacrylate (composé de formule II où R₁ est l'hydrogène).

Les polymères particulièrement préférés sont ceux présentant une viscosité mesurée au viscosimètre BROOKFIELD, mobile 4, à une vitesse de rotation de 100 tours/minutes dans une solution d'eau à 2 % et à une température d'environ 25°C, supérieure ou égale à 1.000 cps (ou 1.000 mPa.s) et plus préférentiellement allant de 5.000 à 40.000 cps (5.000 à 40.000 mPa.s) et plus particulièrement de 6.500 à 35.000 cps (6.500 à 35.000 mPa.s).

Les poly(acide 2-acrylamido 2-méthylpropane sulfonique) réticulés utilisés dans la composition de l'invention peuvent être obtenus selon le procédé de préparation comprenant les étapes suivantes :
(a) on disperse ou on dissout le monomère acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Le poly(acide 2-acrylamido 2-méthylpropane sulfonique) réticulé utilisé dans la composition de l'invention peut être notamment le produit commercialisé par la société Hoechst sous la dénomination commerciale « Hostacerin AMPS » (nom CTFA : ammonium polyacryldimethyltauramide).

Le poly(acide 2-acrylamido 2-méthylpropane sulfonique) réticulé utilisé dans la composition de l'invention est de préférence présent en une quantité allant de 0,1 à 10 % en poids, mieux de 0,2 à 5 % en poids et plus préférentiellement de 0,2 à 2 % en poids par rapport au poids total de la composition.

La composition de l'invention contient au moins un milieu aqueux pouvant constituer le milieu physiologiquement acceptable d'une composition topique. Ce milieu aqueux peut comporter, outre l'eau, éventuellement des additifs tels que les polyols comme la glycérine, les glycols (butylène glycol, propylène glycol, polyéthylène glycols), les sucres et les alcools inférieurs comportant de 1 à 6 et de préférence de 1 à 4 atomes de carbone comme l'éthanol ou l'isopropanol. Quand la composition est sous forme d'émulsion, ce milieu aqueux constitue la phase aqueuse de l'émulsion.

Les compositions selon l'invention peuvent constituer notamment une composition à application topique telle qu'une composition cosmétique ou dermatologique, et elles peuvent se présenter sous toutes les formes galéniques normalement utilisées pour une application topique, en particulier sous forme d'une solution aqueuse éventuellement gélifiée, sous forme de lotions éventuellement biphase, sous forme d'émulsions huile-dans-eau (H/E) ou eau-dans-huile (E/H) ou multiple, ou sous forme d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique. Ces compositions sont préparées selon les méthodes usuelles. Elles peuvent être plus ou moins fluides et avoir l'aspect d'une crème, d'une pommade, d'un lait, d'une lotion, d'un sérum ou d'une mousse. Elles peuvent éventuellement être appliquées sur la peau sous forme d'aérosol. Elles peuvent également se présenter sous forme d'une pâte plus ou moins dure, par exemple sous forme de stick. Elles peut êtres utilisées par exemple comme produit de soin et/ou comme produit de maquillage de la peau.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les séquestrants (EDTA), les agents régulateurs de pH (acide ou base), les bactéricides, les absorbeurs d'odeur, les matières colorantes (pigments, colorants), les sels. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse ou dans la phase aqueuse de la composition. Ces adjuvants ainsi que leurs concentrations doivent être tels qu'ils ne modifient pas la propriété recherchée pour la composition de l'invention.

Comme actifs, on peut citer les vitamines telles que par exemple les vitamines A (rétinol), C (acide ascorbique), B3 ou PP (niacinamide), B5 (panthénol), E (tocophérol), K1, et les dérivés de ces vitamines et notamment leurs esters ; les agents kératolytiques ou prodesquamants tels que les hydroxyacides (α-hydroxy-acides et β-hydroxy-acides) comme l'acide glycolique, l'acide citrique, l'acide lactique, l'acide salicylique et leurs dérivés ; les agents anti-radicaux libres ; les filtres solaires ; les agents hydratants comme les polyols ; les céramides ; les rétinoïdes ; et les polymères tenseurs notamment organiques tels que les latex, les hydrolysats de protéines et les dérivés de chitine ; la DHEA et ses dérivés tels que l'alpha-hydroxy-DHEA ; le coenzyme Q10, et leurs mélanges.

Comme filtres solaires, la composition de l'invention peut comprendre les filtres chimiques UVA et UVB habituellement utilisables dans le domaine cosmétique, y compris les filtres UV organiques insolubles, c'est-à-dire les filtres UV organiques dont la solubilité dans l'eau est inférieure à 0,1 % en poids et dont la solubilité dans l'huile de vaseline est inférieure à 0,1% en poids.

Comme filtres solaires, on peut utiliser aussi les filtres minéraux tels que le dioxyde de titane et l'oxyde de zinc, enrobés ou non.

On peut aussi utiliser un mélange de ces différentes sortes de filtres.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) les filtres actifs dans l'UV-A tels que l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone ;
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle ;
(7) les dérivés siliciés de benzimidazolyl-benzazoles N-substitués ou de benzofuranyl-benzazoles, et en particulier ceux décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

Le pH de la composition selon l'invention peut aller par exemple de 5 à 9, de préférence de 5,5 à 7,5 et mieux de 6 à 7. Le pH peut si nécessaire être ajusté par addition d'acides tels que l'acide chlorhydrique, l'acide citrique, l'acide glycolique et tout acide approprié.

Selon un mode préféré de réalisation de l'invention, la composition se présente sous forme d'une émulsion, et plus particulièrement sous forme d'une émulsion huile-dans-eau.

Quand la composition est une émulsion, elle comporte une phase huileuse. La proportion de la phase huileuse de l'émulsion peut aller par exemple de 5 à 80 % en poids, et de préférence de 5 à 50 % en poids par rapport au poids total de la composition. Les huiles, les émulsionnants et éventuellement les co-émulsionnants utilisés dans la composition sous forme d'émulsion sont choisis parmi ceux classiquement utilisés dans le domaine cosmétique ou dermatologique. L'émulsionnant et éventuellement le co-émulsionnant sont généralement présents dans la composition, en une proportion allant de 0,1 à 30 % en poids, et de préférence de 0,5 à 20 % en poids par rapport au poids total de la composition. L'émulsion peut, en outre, contenir des vésicules lipidiques.

Comme huiles utilisables dans une émulsion selon l'invention, on peut citer les huiles minérales (huile de vaseline), les huiles d'origine végétale (fraction liquide du beurre de karité, huile de tournesol), les huiles d'origine animale (perhydrosqualène), les huiles de synthèse (polyisobutène hydrogénée, esters gras comme le palmitate d'éthyl-2 hexyle, le myristate d'isopropyle, l'isononanoate d'isononyle, l'octanoate de cétéaryle), les huiles de silicone non volatiles ou volatiles (cyclométhicones telles que cyclopentasiloxane) et les huiles fluorées (perfluoropolyéthers).

La phase huileuse peut aussi comprendre un ou plusieurs corps gras autres que les huiles. Comme corps gras, on peut citer par exemple les alcools gras comme l'alcool stéarylique ou l'acool cétylique ; les acides gras ; les cires telles que les cires microcristallines, la cire de jojoba, la cire de lanoline et la cire d'abeille ; les gommes telles que les gommes de silicone (diméthiconol) ; les résines et notamment les résines de silicone telles que la trifluorométhyl C1-4 alkyldimethicone.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt.

Pour les émulsions H/E, on peut citer par exemple comme émulsionnants, les émulsionnants non ioniques tels que les esters d'acides gras et de glycérol oxyalkylénés (plus particulièrement polyoxyéthylénés), les esters d'acides gras et de sorbitan oxyalkylénés (plus particulièrement oxyéthylénés), les esters d'acides gras oxyalkylénés (oxyéthylénés et/ou oxypropylénés), les éthers d'alcools gras oxyalkylénés (oxyéthylénés et/ou oxypropylénés), les esters de sucres comme le stéarate de sucrose, et leurs mélanges, et les émulsionnants anioniques comme par exemple les alkyl éther sulfates tels que le lauryl éther sulfate de sodium, les sulfosuccinates, les iséthionates et leurs mélanges, ainsi que les mélanges des émulsionnants non ioniques et anioniques cités ci-dessus.

Selon un mode préféré de réalisation de l'invention, la composition se présente sous forme d'une émulsion H/E comportant comme émulsionnant, un ou plusieurs éthers polyoxyéthylénés et/ou polyoxypropylénés d'alcools gras tels que l'alcool laurylique ou les mélanges d'alcools gras en C12-C15. On peut citer notamment comme tensioactif de ce type le produit commercialisé sous la dénomination Cosmacol PSE par la société Condea Augusta, comprenant le mélange suivant (noms en CTFA) : Dimyristyl tartrate / cetearyl alcohol / C12-15 Pareth-7 / PPG-25-Laureth-25.

Selon un autre mode préféré de réalisation de l'invention, la composition se présente sous forme d'une émulsion H/E comportant, comme émulsionnant, un ou plusieurs émulsionnants anioniques choisis parmi les alkyl éther sulfates, les sulfosuccinates, les iséthionates et leurs mélanges.

Aussi, l'invention a encore pour objet une émulsion huile-dans-eau comprenant une phase huileuse dispersée dans une phase aqueuse, caractérisée en ce qu'elle comprend au moins un silicate mixte, au moins un polysaccharide et au moins un polymère poly(acide 2-acrylamido 2-méthylpropane sulfonique) réticulé et neutralisé à au moins 90 %, et au moins un émulsionnant choisi parmi les éthers d'alcool gras polyoxyéthylénés et/ou polyoxypropylénés, les alkyl éther sulfates, les sulfosuccinates, les iséthionates et leurs mélanges. De manière préférée, comme éther d'alcool gras polyoxyéthyléné et/ou polyoxypropyléné, on utilise le mélange indiqué ci-dessus.

Appliquée sur la peau, la composition selon l'invention donne un bon effet tenseur. Ainsi, elle peut être utilisée sur la peau comme composition de soin et/ou de maquillage, notamment pour tendre la peau, la lisser et atténuer voire effacer immédiatement les ridules et rides de la peau. On entend par « immédiatement » un effet qui se produit dans 15 à 30 minutes après l'application de la composition.

Aussi, l'invention a également pour objet l'utilisation cosmétique de la composition telle que définie ci-dessus, pour tendre la peau et la lisser en vue d'atténuer immédiatement les rides et/ou ridules.

L'invention a encore pour objet un procédé de traitement cosmétique des rides et/ou ridules de la peau, consistant à appliquer sur la peau une composition telle que définie ci-dessus, en une quantité suffisante pour estomper la ride par effet tenseur. La quantité suffisante doit être telle que la quantité de silicate mixte soit celle indiquée ci-dessus.

L'invention est illustrée plus en détail dans les exemples suivants. Les quantités sont en pourcentage en poids, sauf mention contraire.

### Exemple 1 : Emulsion H/E

| *Phase A :* | |
|---|---|
| Dimyristyl Tartrate / Cetearyl alcohol / C12-15 Pareth-7 / PPG-25-Laureth-25 (Cosmacol PSE) | 1,5 % |
| Huile de silicone | 5 % |
| Huile végétale | 5 % |
| Conservateur | 0,15 % |
| Ethylhexyl Methoxycinnamate (filtre) | 1 % |

| *Phase B :* | |
|---|---|
| Glycérine | 5 % |
| Conservateur | 0,5 % |
| Disodium EDTA (séquestrant) | 5 % |
| Hostacerin AMPS | 0,4 % |

| *Phase C* | |
|---|---|
| Gomme de xanthane | 0,2 % |

| Phase D : | |
|---|---|
| Laponite XLG | 3 % |
| Eau déminéralisée | q. s. 100% |

Mode opératoire : On chauffe séparément les phases A et B à 75°C environ sous agitation, puis on verse la phase A dans la phase B sous agitation. Ensuite, on ajoute la phase C et on arrête le chauffage tout en maintenant l'agitation. On prépare la phase D en saupoudrant la Laponite dans l'eau sous agitation à température ambiante : on obtient un gel transparent incolore assez épais en 15-20 mn et on l'ajoute à l'émulsion obtenue précédemment et refroidie à une température de 40-50°C. On maintient l'agitation jusqu'au retour à température ambiante (environ 20 à 25°C).

On obtient une crème qui a un effet tenseur immédiat, permettant l'estompage des ridules du visage. Cet effet tenseur est mis en évidence par la méthode de mesure de rétraction à l'extensiomètre, décrite ci-dessous :
Le principe consiste à mesurer avant traitement et après traitement la longueur d'une éprouvette de *stratum corneum* isolé et de déterminer le pourcentage de rétraction de l'éprouvette.

On utilise des éprouvettes de 1 cm x 0,4 cm de *stratum corneum,* d'épaisseur allant de 10 à 20 µm disposées sur l'extensiomètre MTT 610 commercialisé par la société DIASTRON. L'éprouvette est placée entre 2 mâchoires puis laissée pendant 12 heures dans une atmosphère à 30°C et 40 % d'humidité relative. On tracte à la vitesse de 2 mm/minute l'éprouvette d'une longueur comprise entre 5 et 10% de la longueur initiale pour déterminer la longueur L₁ à partir de laquelle l'éprouvette commence à exercer une force sur les mâchoires et détectée par l'appareil. On détend ensuite l'éprouvette puis on applique sur le *stratum corneum* 2 mg de l'émulsion de l'exemple 1. Après évaporation totale de la composition, on tracte l'éprouvette dans les mêmes conditions que celles décrites précédemment pour déterminer également la longueur L₂ pour l'éprouvette traitée. Le pourcentage de rétraction est déterminé par le rapport : 100 x (L₂ - L₁)/L₁.

Pour l'émulsion de l'exemple 1, le pourcentage de rétraction de l'éprouvette de stratum cornéum est de -1.6 +/-0.4.

L'effet tenseur est également mis en évidence sur des modèles : on les prend en photo avant l'application de tout produit puis après avoir appliqué l'émulsion selon l'invention et l'on compare les clichés. On constate une nette diminution de la visibilité des rides (particulièrement la patte d'oie et sous les yeux).

## Revendications

1. Composition contenant au moins un silicate mixte, au moins un polysaccharide et au moins un polymère poly(acide 2-acrylamido 2-méthylpropane sulfonique) réticulé et neutralisé à au moins 90 %.

2. Composition selon la revendication 1, **caractérisée par le fait que** le silicate mixte est choisi parmi les montmorillonites, les hectorites, les bentonites, la beidellite, les saponites.

3. Composition selon la revendication 1 ou 2, **caractérisée par le fait que** le silicate mixte est sous forme de poudre dont les particules ont une taille moyenne allant de 5 à 1000 nm.

4. Composition selon l'une quelconque des revendications 1 à 3, **caractérisée par le fait que** le silicate mixte est un phyllosilicate.

5. Composition selon l'une quelconque des revendications 1 à 3, **caractérisée par le fait que** le silicate mixte appartient à la famille des hectorites.

6. Composition selon l'une quelconque des revendications 1 à 3, **caractérise par le fait que** le silicate mixte est une laponite.

7. Composition selon l'une quelconque des revendications précédentes, **caractérisée par le fait que** le silicate mixte est présent en une quantité allant de 0,01 à 10 % en poids et de préférence de 0,05 à 5 % en poids par rapport au poids total de la composition.

8. Composition selon l'une quelconque des revendications précédentes, **caractérisée par le fait que** le polysaccharide est choisi parmi les dérivés cellulosiques ; les galactomannanes et leurs dérivés ; les extraits d'algues ; les exsudats de plantes ; les exsudats de micro-organismes, et leurs mélanges.

9. Composition selon l'une quelconque des revendications précédentes, **caractérisée par le fait que** le polysaccharide est la gomme de xanthane.

10. Composition selon l'une quelconque des revendications précédentes, **caractérisée par le fait que** le polysaccharide est présent en une quantité allant de 0,01 à 8 % en poids et de préférence de 0,05 à 5 % en poids par rapport au poids total de la composition.

11. Composition selon l'une quelconque des revendications précédentes, **caractérisée par le fait que** le polymère poly(acide 2-acrylamido 2-méthylpropane sulfonique) comprend, distribués de façon aléatoire :
a) de 90 à 99,9% en poids de motifs de formule générale (I) suivante : 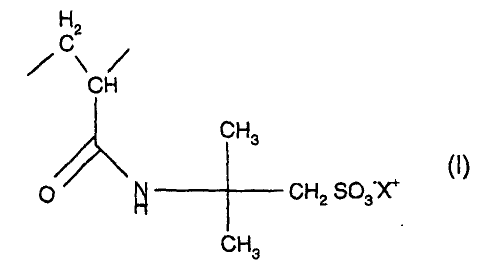 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.

12. Composition selon la revendication précédente, **caractérisée par le fait que** le polymère poly(acide 2-acrylamido 2-méthylpropane sulfonique) comprend de 98 à 99,5 % en poids de motifs de formule (I) et de 0,2 à 2 % en poids de motifs réticulants.

13. Composition selon la revendication 11 ou 12, **caractérisée par le fait que** dans la formule (I) le cation X⁺ est NH₄⁺ .

14. Composition selon l'une quelconque des revendications 11 à 13, **caractérisée par le fait que** les monomères réticulants répondent à la formule générale (II) suivante : 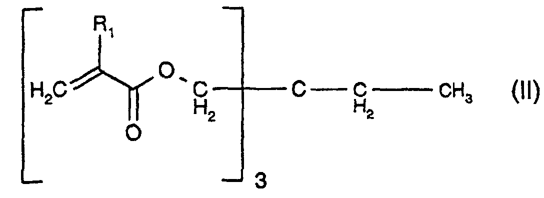 dans laquelle R₁ désigne hydrogène ou un alkyle en C₁-C₄.

15. Composition selon l'une quelconque des revendications 11 à 14, **caractérisée par le fait que** le poly(acide 2-acrylamido 2-méthylpropane sulfonique) est réticulé par le triméthylol propane triacrylate.

16. Composition selon l'une quelconque des revendications précédentes, **caractérisée par le fait que** la quantité de poly(acide 2-acrylamido 2-méthylpropane sulfonique) réticulé va de 0,1 à 10 % en poids par rapport au poids total de la composition.

17. Composition selon l'une quelconque des revendications précédentes **caractérisée par le fait qu'**elle comporte un milieu aqueux physiologiquement acceptable.

18. Composition selon l'une quelconque des revendications précédentes, **caractérisée par le fait qu'**elle contient en outre un ou plusieurs principes actifs choisis parmi les vitamines, les agents kératolytiques ou prodesquamants, les agents anti-radicaux libres, les filtres solaires, les agents hydratants, les céramides, les rétinoïdes et les polymères tenseurs, la DHEA et ses dérivés, le coenzyme Q10.

19. Composition selon l'une quelconque des revendications précédentes, **caractérisée par le fait qu'**elle a un pH allant de 5 à 9.

20. Composition selon l'une quelconque des revendications précédentes, **caractérisée par le fait qu'**elle se présente sous forme d'une émulsion.

21. Composition selon l'une quelconque des revendications précédentes **caractérisée par le fait qu'**elle est sous forme d'émulsion H/E.

22. Composition selon la revendication précédente **caractérisée par le fait qu'**elle contient au moins un émulsionnant choisi parmi les émulsionnants non ioniques, les émulsionnants anioniques et leurs mélanges.

23. Emulsion huile-dans-eau comprenant une phase huileuse dispersée dans une phase aqueuse, **caractérisée par le fait qu'**elle comprend au moins un silicate mixte, au moins un polysaccharide, au moins un polymère poly(acide 2-acrylamido 2-méthylpropane sulfonique) réticulé et neutralisé à au moins 90 %, et au moins un émulsionnant choisi parmi les éthers d'alcool gras polyoxyéthylénés et/ou polyoxypropylénés, les alkyl éther sulfates, les sulfosuccinates, les iséthionates et leurs mélanges.

24. Emulsion selon la revendication précédente, **caractérisée par le fait que** l'émulsionnant est un mélange de Dimyristyl tartrate / cetearyl alcohol / C12-15 Pareth-7 / PPG-25-Laureth-25.

25. Utilisation cosmétique de la composition selon l'une quelconque des revendications 1 à 22 ou de l'émulsion selon l'une quelconque des revendications 23 et 24, pour tendre la peau et la lisser en vue d'atténuer immédiatement les rides et/ou ridules.

26. Procédé de traitement cosmétique des rides et/ou ridules de la peau, consistant à appliquer sur la peau, une composition selon l'une quelconque des revendications 1 à 22 ou une émulsion selon l'une quelconque des revendications 23 et 24, en une quantité suffisante pour estomper la ride ou la ridule par effet tenseur.

## Patentansprüche

1. Zusammensetzung, die mindestens ein Mischsilicat, mindestens ein Polysaccharid und mindestens ein vernetztes und zumindest zu 90 % neutralisiertes Poly(2-acrylamido-2-methylpropansulfonsäure)-Polymer enthält.

2. Zusammensetzung nach Anspruch 1, **dadurch gekennzeichnet, dass** das Mischsilicat unter den Monomorilloniten, Hectoriten, Bentoniten, Beidellit und Saponiten ausgewählt ist.

3. Zusammensetzung nach Anspruch 1 oder 2, **dadurch gekennzeichnet, dass** das Mischsilicat als Pulver vorliegt, dessen Partikel eine mittlere Größe von 5 bis 1 000 nm aufweisen.

4. Zusammensetzung nach einem der Ansprüche 1 bis 3, **dadurch gekennzeichnet, dass** das Mischsilicat ein Phyllosilicat ist.

5. Zusammensetzung nach einem der Ansprüche 1 bis 3, **dadurch gekennzeichnet, dass** das Mischsilicat aus der Gruppe der Hectorite stammt.

6. Zusammensetzung nach einem der Ansprüche 1 bis 3, **dadurch gekennzeichnet, dass** das Mischsilicat ein Laponit ist.

7. Zusammensetzung nach einem der vorhergehenden Ansprüche, **dadurch gekennzeichnet, dass** das Mischsilicat in einer Menge von 0,01 bis 10 Gew.-% und vorzugsweise 0,05 bis 5 Gew.-%, bezogen auf das Gesamtgewicht der Zusammensetzung, vorliegt.

8. Zusammensetzung nach einem der vorhergehenden Ansprüche, **dadurch gekennzeichnet, dass** das Polysaccharid unter den Cellulosederivaten; Galactomannanen und deren Derivaten; Algenextrakten; Pflanzenexsudaten; Exsudaten von Mikroorganismen und deren Gemischen ausgewählt ist.

9. Zusammensetzung nach einem der vorhergehenden Ansprüche, **dadurch gekennzeichnet, dass** es sich bei dem Polysaccharid um Xanthangummi handelt.

10. Zusammensetzung nach einem der vorhergehenden Ansprüche, **dadurch gekennzeichnet, dass** das Polysaccharid in einer Menge von 0,01 bis 8 Gew.-% und vorzugsweise 0,05 bis 5 Gew.-%, bezogen auf das Gesamtgewicht der Zusammensetzung, vorliegt.

11. Zusammensetzung nach einem der vorhergehenden Ansprüche, **dadurch gekennzeichnet, dass** das Poly(2-acrylamido-2-methylpropansulfonsäure)-Polymer zufällig verteilt enthält:
a) 90 bis 99,9 Gew.-% Einheiten der folgenden allgemeinen Formel (I): 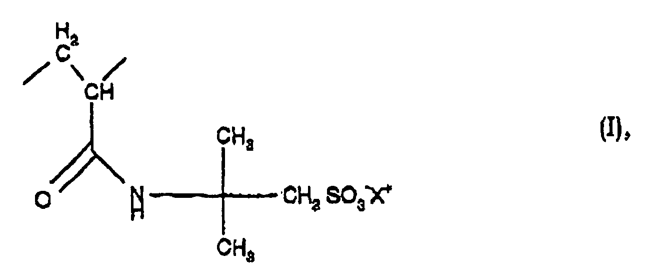 worin X⁺ ein Kation oder ein Gemisch von Kationen bedeutet, wobei höchstens 10 Mol-% der Kationen X⁺ Protonen H⁺ sein können; und
b) 0,01 bis 10 Gew.-% Vernetzungseinheiten, die von mindestens einem Monomer stammen, das mindestens zwei olefinischen Doppelbindungen aufweist; wobei die Gewichtsanteile bezogen auf das Gesamtgewicht des Polymers definiert sind.

12. Zusammensetzung nach dem vorhergehenden Anspruch, **dadurch gekennzeichnet, dass** das Poly(2-acrylamido-2-methylpropansulfonsäure)-Polymer 98 bis 99,5 Gew.-% Einheiten der Formel (I) und 0,2 bis 2 Gew.-% Vernetzungseinheiten enthält.

13. Zusammensetzung nach Anspruch 11 oder 12, **dadurch gekennzeichnet, dass** in der Formel (I) das Kation X⁺ NH₄⁺ bedeutet.

14. Zusammensetzung nach einem der Ansprüche 11 bis 13, **dadurch gekennzeichnet, dass** die Vernetzungsmonomere der folgenden allgemeinen Formel (II) entsprechen: 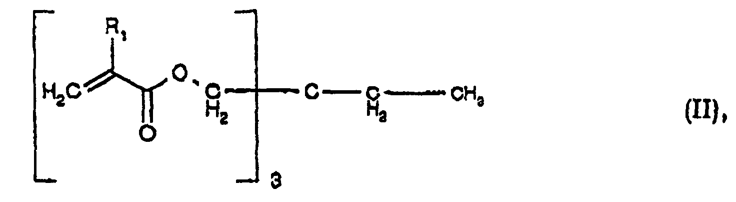 worin Ri Wasserstoff oder C₁₋₄-Alkyl bedeutet.

15. Zusammensetzung nach einem der Ansprüche 11 bis 14, **dadurch gekennzeichnet, dass** die Poly(2-acrylamido-2-methylpropansulfonsäure) mit Trimethylolpropantriacrylat vernetzt ist.

16. Zusammensetzung nach einem der vorhergehenden Ansprüche, **dadurch gekennzeichnet, dass** der Mengenanteil der vernetzten Poly(2-acrylamido-2-methylpropansulfonsäure) im Bereich von 0,1 bis 10 Gew.-%, bezogen auf das Gesamtgewicht der Zusammensetzung, liegt.

17. Zusammensetzung nach einem der vorhergehenden Ansprüche, **dadurch gekennzeichnet, dass** sie ein physiologisch akzeptables wässriges Medium enthält.

18. Zusammensetzung nach einem der vorhergehenden Ansprüche, **dadurch gekennzeichnet, dass** sie ferner einen oder mehrere Wirkstoffe enthält, die unter den Vitaminen, Keratolytika oder abschuppungsfördernden Wirkstoffen, Radikalfängern für freie Radikale, Sonnenschutzfiltern, Hydratisierungsmitteln, Ceramiden, Retinoiden und straffenden Polymeren, DHEA und seinen Derivaten und Coenzym Q10 ausgewählt sind.

19. Zusammensetzung nach einem der vorhergehenden Ansprüche, **dadurch gekennzeichnet, dass** sie einen pH-Wert von 5 bis 9 aufweist.

20. Zusammensetzung nach einem der vorhergehenden Ansprüche, **dadurch gekennzeichnet, dass** sie als Emulsion vorliegt.

21. Zusammensetzung nach einem der vorhergehenden Ansprüche, **dadurch gekennzeichnet, dass** sie als O/W-Emulsion vorliegt.

22. Zusammensetzung nach dem vorhergehenden Anspruch, **dadurch gekennzeichnet, dass** sie mindestens einen Emulgator enthält, der unter den nichtionischen Emulgatoren, anionischen Emulgatoren und deren Gemischen ausgewählt ist.

23. Öl-in-Wasser-Emulsion, die eine in einer wässrigen Phase dispergierte Ölphase aufweist, **dadurch gekennzeichnet, dass** sie mindestens ein Mischsilicat, mindestens ein Polysaccharid, mindestens ein vernetztes und zumindest zu 90 % neutralisiertes Poly(2-acrylamido-2-methylpropansulfonsäure)-Polymer und mindestens einen Emulgator enthält, der unter den polyethoxylierten und/oder polypropoxylierten Fettalkoholethern, Alkylethersulfaten, Sulfosuccinaten, Isethionaten und deren Gemischen ausgewählt ist.

24. Emulsion nach dem vorhergehenden Anspruch, **dadurch gekennzeichnet, dass** es sich bei dem Emulgator um ein Gemisch von Dimyristyl Tartrate / Cetearyl Alcohol / C12-15 Pareth-7 / PPG-25-Laureth-25 handelt.

25. Kosmetische Verwendung der Zusammensetzung nach einem der Ansprüche 1 bis 22 oder der Emulsion nach einem der Ansprüche 23 bis 24, um die Haut zu straffen und zu glätten, um sofort die Falten und/ oder Fältchen abzuschwächen.

26. Verfahren zur kosmetischen Behandlung von Falten und/oder Fältchen der Haut, das darin besteht, auf die Haut eine Zusammensetzung nach einem der Ansprüche 1 bis 22 oder eine Emulsion nach einem der Ansprüche 23 bis 24 in einer Menge aufzutragen, die ausreichend ist, um die Falten oder Fältchen durch eine Straffungswirkung zu mildern.

## Claims

1. Composition containing at least one mixed silicate, at least one polysaccharide and at least one crosslinked poly(2-acrylamido-2-methylpropanesulphonic acid) polymer which is at least 90% neutralized.

2. Composition according to Claim 1, **characterized in that** the mixed silicate is chosen from montmorillonites, hectorites, bentonites, beidellite and saponites.

3. Composition according to Claim 1 or 2,
**characterized in that** the mixed silicate is in the form of a powder whose particles have a mean size ranging from 5 to 1 000 nm.

4. Composition according to any one of Claims 1 to 3, **characterized in that** the mixed silicate is a phyllosilicate.

5. Composition according to any one of Claims 1 to 3, **characterized in that** the mixed silicate belongs to the hectorite family.

6. Composition according to any one of Claims 1 to 3, **characterized in that** the mixed silicate is a laponite.

7. Composition according to any one of the preceding claims, **characterized in that** the mixed silicate is present in a quantity ranging from 0.01 to 10% by weight and preferably from 0.05 to 5% by weight relative to the total weight of the composition.

8. Composition according to any one of the preceding claims, **characterized in that** the polysaccharide is chosen from cellulose derivatives; galactomannans and their derivatives; algal extracts; plant exudates; microbial exudates, and mixtures thereof.

9. Composition according to any one of the preceding claims, **characterized in that** the polysaccharide is xanthan gum.

10. Composition according to any one of the preceding claims, **characterized in that** the polysaccharide is present in a quantity ranging from 0.01 to 8% by weight and preferably from 0.05 to 5% by weight relative to the total weight of the composition.

11. Composition according to any one of the preceding claims, **characterized in that** the poly(2-acrylamido-2-methylpropanesulphonic acid) polymer comprises, randomly distributed:
a) from 90 to 99.9% by weight of units having the following general formula (I): 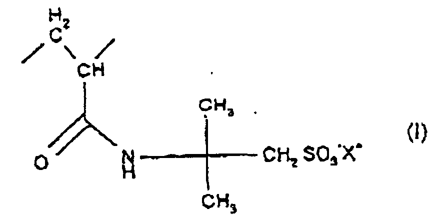 in which X⁺ denotes a cation or a mixture of cations, it being possible for at most 10 mol% of the cations X⁺ to be protons H⁺;
b) from 0.01 to 10% by weight of crosslinking units obtained from at least one monomer having at least two olefin double bonds, the proportions by weight being defined relative to the total weight of the polymer.

12. Composition according to the preceding claim, **characterized in that** the poly(2-acrylamido-2-methylpropanesulphonic acid) polymer comprises from 98 to 99.5% by weight of units of formula (I) and from 0.2 to 2% by weight of crosslinking units.

13. Composition according to Claim 11 or 12, **characterized in that** in formula (I), the cation X⁺ is NH₄⁺.

14. Composition according to any one of Claims 11 to 13, **characterized in that** the crosslinking monomers correspond to the following general formula (II): 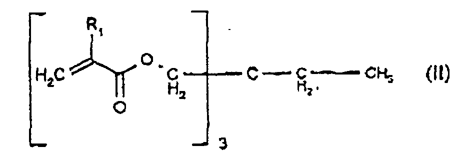 in which R₁ denotes hydrogen or a C₁-C₄ alkyl.

15. Composition according to any one of Claims 11 to 14, **characterized in that** the poly(2-acrylamido-2-methylpropanesulphonic acid) is crosslinked with trimethylolpropane triacrylate.

16. Composition according to any one of the preceding claims, **characterized in that** the quantity of crosslinked poly(2-acrylamido-2-methylpropanesulphonic acid) ranges from 0.1 to 10% by weight relative to the total weight of the composition.

17. Composition according to any one of the preceding claims, **characterized in that** it comprises a physiologically acceptable aqueous medium.

18. Composition according to any one of the preceding claims, **characterized in that** it contains, in addition, one or more active ingredients chosen from vitamins, keratolytic or prodesquamating agents, anti-free radical agents, sunscreens, moisturizing agents, ceramides, retinoids and tightening polymers, DHEA and its derivatives, coenzyme Q10.

19. Composition according to any one of the preceding claims, **characterized in that** it has a pH ranging from 5 to 9.

20. Composition according to any one of the preceding claims, **characterized in that** it is provided in the form of an emulsion.

21. Composition according to any one of the preceding claims, **characterized in that** it is in the form of an O/W emulsion.

22. Composition according to the preceding claim, **characterized in that** it contains at least one emulsifier chosen from nonionic emulsifiers, anionic emulsifiers and mixtures thereof.

23. Oil-in-water emulsion comprising an oily phase dispersed in an aqueous phase, **characterized in that** it comprises at least one mixed silicate, at least one polysaccharide, at least one crosslinked poly(2-acrylamido-2-methylpropanesulphonic acid) polymer which is at least 90% neutralized, and at least one emulsifier chosen from polyoxyethylenated and/or polyoxypropylenated fatty alcohol ethers, alkyl ether sulphates, sulphosuccinates, isethionates and mixtures thereof.

24. Emulsion according to the preceding claim, **characterized in that** the emulsifier is a mixture of dimyristyl tartrate/cetearyl alcohol/C12-15 Pareth-7/PPG-25-Laureth-25.

25. Cosmetic use of the composition according to any one of Claims 1 to 22 or of the emulsion according to either of Claims 23 and 24, for tightening the skin and smoothing it so as to immediately attenuate wrinkles and/or fine lines.

26. Method for the cosmetic treatment of wrinkles and/or fine lines on the skin, consisting in applying to the skin a composition according to any one of Claims 1 to 22 or an emulsion according to either of Claims 23 and 24, in a sufficient quantity to blur the wrinkle or fine line by a tightening effect.
